(19) Europäisches Patentamt
European Patent Office
Office européen des brevets

(11) Veröffentlichungsnummer: **0 157 315**
**B1**

(12) # EUROPÄISCHE PATENTSCHRIFT

(45) Veröffentlichungstag der Patentschrift: **01.06.88**

(51) Int. Cl.⁴: **C 07 C 91/26,** C 07 C 121/453

(21) Anmeldenummer: **85103421.5**

(22) Anmeldetag: **22.03.85**

(54) **Optisch aktives Di-(3-chlor-2-oxy-propyltrimethylammonium)-tartrat.**

(30) Priorität: **04.04.84 CH 1704/84**

(43) Veröffentlichungstag der Anmeldung:
**09.10.85 Patentblatt 85/41**

(45) Bekanntmachung des Hinweises auf die Patenterteilung:
**01.06.88 Patentblatt 88/22**

(84) Benannte Vertragsstaaten:
**AT BE CH DE FR GB IT LI LU NL SE**

(56) Entgegenhaltungen:
**EP - A - 0 005 223**

(73) Patentinhaber: **LONZA AG, Gampel/Wallis (CH)**

(72) Erfinder: **Tenud, Leander, Dr., Balfrinstrasse 23, CH-Visp (CH)**
Erfinder: **Gosteli, Jacques, Dr., Anwilerstrasse 10, CH-Basel (CH)**

(74) Vertreter: **Weinhold, Peter, Dr. et al, Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8, D-8000 München 40 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 157 315 B1

**Beschreibung**

Die vorliegende Erfindung betrifft optisch aktives Di-[3-chlor-2-oxy-propyltrimethylammonium]-tartrat, insbesondere Di-[(–)-3-chlor-2-oxy-propyltrimethylammonium]-L-(+)-tartrat (in der Folge COP-tartrat genannt), ein Verfahren zur Herstellung desselben und die Anwendung desselben zur Herstellung von optisch aktivem Carnitinnitrilchlorid.

Da die Racematspaltung des freien Carnitins infolge der geringen Stabilität der freien Säuregruppe Schwierigkeiten bereitet, hat man zur Racematspaltung vornehmlich die Nitrile oder die Amide des Carnitins eingesetzt.

Z.B. ist es aus der DD-Patentschrift 23217 bekannt, Carnitinnitrilchlorid, das durch Behandeln mit Silberoxid in das Hydroxid oder durch Behandeln mit Silbercarbonat in das Carbonat übergeführt wurde, mit einer optisch aktiven Säure in die Diastereomere überzuführen, von welchen das geeignete Diastereomer abgetrennt und daraus das gewünschte Carnitinderivat isoliert. Ein anderer Weg aus BE-PS 660 039 geht von Carnitinamidhydrochlorid aus, welches mit Camphersäure in Gegenwart von $AgNO_3$ zum Diastereomergemisch umgesetzt wird. Das geeignete Diastereomer wird wiederum abgetrennt und zerlegt.

Obige Verfahren weisen aber erhebliche Nachteile auf. Darunter fallen die in grossen Mengen anfallenden, schwer abtrennbaren Salzverunreinigungen, die die Racematspaltung erschweren, sowie die zahlreichen Verfahrensschritte, die zur Erreichung des zur Racematspaltung zugänglichen Carnitinamids resp. Carnitinnitrils erforderlich sind und somit eine technische Anwendung zu kostenaufwendig machen. Diese Schwierigkeiten werden noch vergrössert, da durch die Anwendung von Silbersalzen unter Lichtausschluss gearbeitet werden muss, um Schwärzungen des Reaktionsgutes zu vermeiden.

Aufgabe der vorliegenden Erfindung ist es, diese Nachteile zu beseitigen, und ein Verfahren vorzuschlagen, das es erlaubt, auf einfache Weise optisch aktives Carnitinnitrilchlorid, insbesondere das (–)-Carnitinnitrilchlorid zu gewinnen. Dies wird durch Verwendung von optisch aktivem Di-[3-chlor-2-oxy-propyltrimethylammonium]-tartrat gemäss Patentanspruch 1 erreicht. Zur Herstellung von (–)-Carnitinnitrilchlorid wird Di-[(–)-3-chlor-2-oxy-propyltrimethylammonium]-L-(+)-tartrat (COP-tartrat) verwendet. Diese Verbindungen sind neu und haben nachstehende Formel:

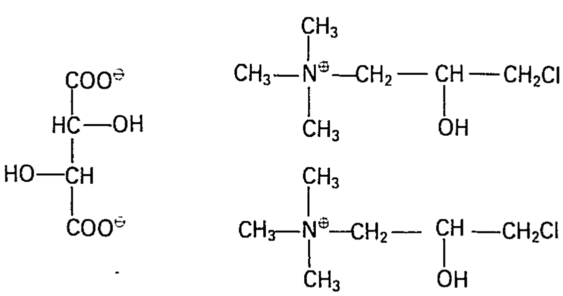

Das COP-tartrat kann nach verschiedenen Methoden hergestellt werden. Bevorzugt wird das COP-tartrat entweder durch Umsetzung von racemischem 3-Chlor-2-oxy-propyltrimethylammonium-chlorid mit L-(+)-Weinsäure zweckmässig in Gegenwart von Trialkylaminen oder durch Umsetzung von L-(+)-Weinsäure mit Trimethylamin und anschliessendem Umsatz mit Epichlorhydrin hergestellt.

Wird nach der ersten Methode vorgegangen, wird zunächst das racemische 3-Chlor-2-oxy-propyltrimethylammonium-chlorid aus Epichlorhydrin und Trimethylamin hergestellt und dieses mit L-(+)-Weinsäure in Gegenwart von Trialkylaminen in das COP-tartrat übergeführt.

Als Trialkylamine kommen vorzugsweise solche mit 2–12 C-Atomen in Frage. Solche sind Triethylamin, Tributylamin, Tripropylamin, Tripentylamin, Trioctylamin. Vorzugsweise wird Tributylamin verwendet.

Das COP-tartrat kann von seinem Diastereomer durch Kristallisation abgetrennt werden.

Eine bevorzugte Ausführungsform zur Herstellung des COP-tartrats gemäss der Erfindung wird im folgenden beschrieben: Ausgehend von 1 Mol rechtsdrehender Weinsäure werden zur Herstellung des Diastereomergemisches zweckmässig 1,6 bis 3 Mol, vorzugsweise 1,8 bis 2,5 Mol Tri-n-butylamin mit zweckmässig 1,6 bis 3 Mol, vorzugsweise 1,8 bis 2,2 Mol, racemisches 3-Chlor-2-oxy-propyltrimethylammonium-chlorid umgesetzt. Man arbeitet vorzugsweise in Gegenwart von Wasser und/oder einem mit Wasser nicht mischbaren Lösungsmittel, wie Methylenchlorid, Chloroform, und bei Temperaturen von 0 bis 30 °C, vorzugsweise 15 bis 25 °C.

Nach Abtrennung des Tri-n-alkylaminhydrochlorids durch Extraktion mit inerten Lösungsmitteln, wie Methylenchlorid, Chloroform, wird das gewünschte Isomer durch fraktionierte Kristallisation, nach Eindampfen der wässrigen Phase, unter vermindertem Druck isoliert.

Zweckmässig wird das Diastereomergemisch in Lösungsmitteln wie Wasser oder niedrigen Alkoholen, wie Methanol, Ethanol, vorzugsweise in Methanol, gelöst.

Die Kristallisation des gewünschten Isomers, des COP-tartrats, wird dadurch erreicht, dass man zweckmässig ein Verdünnungsmittel, vorzugsweise Aceton, zugibt.

Nach einer weiteren Methode wird so vorgegangen, dass zunächst L-(+)-Weinsäure, gelöst in Wasser oder suspendiert in Alkohol, zweckmässig Methanol oder Ethanol, vorgelegt wird, anschliessend mit Trimethylamin neutralisiert und das als Zwischenprodukt gebildete Di-[trimethylammonium]-tartrat mit Epichlorhydrin bei Temperaturen von 17 bis 30 °C in das gewünschte COP-tartrat und dessen Diastereomere übergeführt wird.

Eine bevorzugte Ausführungsform zur Herstellung des COP-tartrats gemäss der Erfindung wird im folgenden beschrieben: Ausgehend von 1 Mol L-(+)-Weinsäure, gelöst in 200 bis 250 g Wasser oder suspendiert in niederen Alkoholen, werden bei Temperaturen von 0 bis 30 °C 1,6 bis 2,5 Mol

Trimethylamin, vorzugsweise 1,8 bis 2,1 Mol Trimethylamin zugesetzt. Der pH der Lösung liegt zweckmässig bei 6,5 bis 7,5. Anschliessend werden zweckmässig 1,6 bis 3 Mol Epichlorhydrin zugesetzt und die Temperatur auf 15 bis 30 °C, vorzugsweise 20 bis 28 °C, gehalten.

Wird mit Wasser gearbeitet, bildet sich eine flüssige Phase aus. Nach Abdampfen des Wassers, zweckmässig im Vakuum, resultiert ein öliger Rückstand, aus dem durch Behandeln mit organischen Lösungsmitteln, zweckmässig mit Methanol/Aceton, das gewünschte COP-tartrat auskristallisiert wird. Wird mit Alkoholen, z.B. Methanol, Ethanol, gearbeitet, fällt das gewünschte COP-tartrat aus und kann abgetrennt werden.

Eine weitere Methode zur Herstellung des COP-tartrats besteht darin, dass man zunächst das Silbersalz der Weinsäure mit Silbernitrat und Alkalitartrat herstellt und dieses Silbertartrat in Wasser suspendiert und mit racemischem 3-Chlor-2-oxy-propyltrimethylammonium-chlorid umsetzt.

Das gewünschte COP-tartrat kann durch Kristallisation gewonnen, bzw. vom diastereomeren Salz abgetrennt werden.

Das Di-[(–)-3-chlor-2-oxy-propyltrimethylammonium]-L-(+)-tartrat der Erfindung hat nachstehende Eigenschaften:

Smp. 159 °C (nach Umkristallisation aus Methanol/Aceton)

$[\alpha]^{24}_{D} = -10{,}8°$ (c = 1,04 in Wasser)

pH der Lösung (1%): 7

Analyse: C ber. 42,39% gef. 42,36%
(521.1.4) H ber. 7,56% gef. 7,99%
N ber. 6,18% gef. 6,36%

IR (KBr) Spektrum: 3,15, 6,30, 7,20, 9,15, 10,35 Mikron.

Zur Herstellung von Di-[(+)-chlor-2-oxy-propyltrimethylammonium]-D-(–)-tartrats wird die Racematspaltung mit Hilfe von D-(–)-Weinsäure durchgeführt.

Smp. 159 °C (nach Umkristallisation aus Methanol/Aceton.

$[\alpha]^{24}_{D} = +10{,}8°$ (c = 1,04 in Wasser).

Durch die erfindungsgemässen Verfahren erfolgt die Racematspaltung sehr früh. Es ist somit möglich, für die weiteren Schritte bis zum Carnitinnitrilchlorid und Carnitin nur noch mit einem Antipoden zu arbeiten, wodurch eine Belastung der weiteren Reaktionen durch den anderen Antipoden entfällt. Es war nicht ohne weiteres vorauszusehen, dass bei der Nachfolgereaktion, die letztlich bis zum Carnitin führt, keine weitere Racemisierung auftritt.

Das erfindungsgemässe Di-[(–)-3-chlor-2-oxy-propyltrimethylammonium]-L-(+)-tartrat (COP-tartrat) kann auf einfache Weise in das (–)-Carnitinnitrilchlorid und (–)-Carnitin übergeführt.

Dabei wird entweder das COP-tartrat zunächst mit $CaCl_2$ umgesetzt, das Ca-tartrat abgetrennt und das (–)-3-Chlor-2-oxy-propyltrimethylammonium-chlorid isoliert. Letzteres kann mit Alkalicyaniden in das (–)-Carnitinnitrilchlorid übergeführt werden. Als Alkalicyanide kommen LiCN, KCN, NaCN, vorzugsweise NaCN, zum Einsatz.

Man kann aber auch die Zerlegung, d.h. doppelte Salzumsetzung, des COP-tartrats und die Cyanid-Substitutionsreaktion auch in einer Stufe durchführen. Dann setzt man zweckmässig Erdalkalicyanide, vorzugsweise $Ca(CN)_2$ ein. Dabei fällt die Weinsäure als Ca-Salz aus, und das (–)-Carnitinnitrilchlorid kann aus der Reaktionslösung isoliert werden.

Vorzugsweise wird die Freisetzung bzw. Reaktion, gleich welche Methode zur Anwendung kommt, in Wasser als Lösungsmittel, durchgeführt.

Nach einer weiteren Methode der Erfindung kann das nach der Spaltung des COP-tartrats isolierte optisch aktive 3-Chlor-2-oxy-propyltrimethylammonium-chlorid durch Behandeln mit starken Basen, wie Alkalihydroxid, Alkalialkoholat, Alkali-tert-butylat, in das (–)-Glycidyltrimethylammonium-chlorid übergeführt werden und letzteres durch Behandeln mit Acetoncyanhydrin oder Blausäure in das L-Carnitinnitrilchlorid übergeführt werden.

Diese Methode wird bevorzugt in Alkoholen als Lösungsmittel, bei Temperaturen um Raumtemperatur, durchgeführt.

Die Reinigung des Produktes kann wirksam durch einfache Kristallisation aus Lösungsmitteln, wie niederen Alkoholen, erreicht werden.

Man erhält dadurch Produkte mit optischen Reinheiten von 98+.

Nach diesem Verfahren kann aber auch das Di-[(+)-3-chlor-2-oxy-propyltrimethylammonium]-D-(–)-tartrat in das entsprechende (+)-Carnitinnitrilchlorid hergestellt werden.

Beispiel 1

Herstellung von Di-[3-chlor-2-oxy-propyltrimethylammonium]-tartrat

Zu 7,50 g (50 mMol) L-(+)-Weinsäure, gelöst in 50 ml Wasser, wurden unter Rühren 18,54 g (100 mMol) Tri-n-butylamin getropft, wobei sich die Lösung auf 30 °C erwärmte. Anschliessend wurden 18,81 g (100 mMol) 3-Chlor-2-oxy-propyltrimethylammoniumchlorid, gelöst in 100 ml Wasser, eingetragen. Die klare Lösung wurde mit 8 Portionen à 150 ml Methylenchlorid extrahiert und die Extrakte im Vakuum verdampft; erhalten wurden 21,65 g (97,6% Ausbeute) Tributylamin-hydrochlorid (die letzten beiden Extrakte enthielten zusammen nur noch 0,14 g Material).

Die wässrige Schicht wurde im Rotationsverdampfer zur Trockene eingedampft; es resultierten 23,22 g eines sehr viskosen Öls (102,5%). Dieses wurde in 30 ml Methanol heiss gelöst, langsam mit 93 ml Aceton bis zur Trübung versetzt und letztere durch Zusatz einiger Tropfen Methanol wieder zum Verschwinden gebracht. Nach 72 h wurde die Mutterlauge abdekantiert, die Kristallkruste mit Aceton/Methanol = 3:1 gewaschen und im Vakuum getrocknet.

Ausbeute: 7,20 g Kristalle (31,8% bzw. 63,6% der Theorie), Smp. 147–170 °C.

Das rohe Tartrat wurde in 10 g heissem Methanol gelöst und allmählich 45 ml Aceton zugesetzt, wobei die Kristallisation sofort einsetzte. Über

Nacht wurde das Kristallisationsgefäss im Kühlschrank aufbewahrt, die Mutterlauge abdekantiert, der Kristallkuchen mit Aceton gewaschen und getrocknet.

Es resultierten 5,78 g Kristalle entsprechend 51% der Theorie.

Smp. 150–152 °C.

$[\alpha]_D^{24} = -7,5°$ (c = 1,04 in Wasser)

Aus dem Methylenchloridrückstand (rohes Tributylaminhydrochlorid) wurde durch Lösen in Methylenchlorid, Schütteln mit 1n Natronlauge und Entfernen des Lösungsmittels im Vakuum das Tributylamin in einer Ausbeute von 96% zurückgewonnen.

Beispiel 2

Herstellung von Di-[(3)-chlor-2-oxy-propyltrimethylammonium]-L-(+)-tartrat

Zu 18,75 g (125 mMol) L-(+)-Weinsäure, gelöst in 30 ml Wasser, wurde unter Rühren 39 ml (259 mMol) Trimethylamin innerhalb 10 min zugetropft. Die Temperatur wurde auf 30 °C gehalten. Der pH der Lösung war 7. Anschliessend wurde auf 15 °C gekühlt und unter Rühren 23,15 g (250 mMol) Epichlorhydrin zugetropft. Die Reaktionstemperatur wurde auf 25 °C g ehalten und weitergerührt, bis das Gemisch nur noch aus einer flüssigen Phase bestand.

Nach beendeter Reaktion wurde unter Vakuum (Rotavap) bei 40 °C das Wasser abgedampft. Es resultierten 59,5 g eines viskosen Öls.

Dieser Rückstand wurde in 40 ml heissem Methanol gelöst und allmählich 135 ml Aceton bis zur Trübung zugesetzt. Nach Stehenlassen während 72 h bei Raumtemperatur wurde die Mutterlauge abgegossen und die Kristalle mit Aceton/Methanol (4:1) gewaschen und im Vakuum getrocknet.

Es resultierten 4,75 g plattenförmige Kristalle (Ausbeute: 16,8% d.Th.).

Smp. 150 bis 152 °C

$[\alpha]_D^{24} = -8,1°$ (c = 1 in Wasser)

Beispiel 3

Herstellung von Di-[(–)-3-chlor-2-oxy-propyltrimethylammonium]-L-(+)-tartrat

150 g (1 Mol) L-(+)-Weinsäure wurden in 200 g Methanol suspendiert und bei einer Temperatur von 20 °C wurden innerhalb einer Stunde 106,2 g (1,8 Mol) Trimethylamin und 250 g Ethanol zugesetzt. Die Temperatur wurde auf 20 °C gehalten. Die Weinsäure wurde unter Bildung von Di-trimethylammonium-L-(+)-tartrat gelöst. Anschliessend wurden 166,5 g (1,8 Mol) Epichlorhydrin zugesetzt und die Temperatur bei 20 °C gehalten. Es wurde unter Einhaltung dieser Temperatur 2 Tage weitergerührt. Die ausfallenden Kristalle wurden abfiltriert und mit Aceton/Methanol (4:1) gewaschen und im Vakuum getrocknet.

Das in einer Ausbeute von 38,9% (77,8% d.Th.) erhaltene Produkt hatte einen Smp. von 157 bis 158 °C

$[\alpha]_D^{24} = -9,1°$ (c = 1 in Wasser)

Beispiel 4

Herstellung von Di[(–)-3-chlor-2-oxy-propyltrimethylammonium]-D-(–)-tartrat

46,25 g (127 mMol) Di-Silber-L-(+)-tartrat wurden in 350 ml Wasser suspendiert und mit einer Lösung von 3-Chlor-2-oxy-propyltrimethylammonium-chlorid, gelöst in Wasser, versetzt. Die Suspension wurde 4 h gerührt und das gebildete Silberchlorid abgenutscht und (zwecks schneller Trocknung) mit Methanol und Ether gewaschen und getrocknet. Es resultierten 36,21 g Silberchlorid (99,5% d.Th.).

Das Filtrat wurde im Rotationsverdampfer vollständig eingedampft. Der Rückstand wog nach Trocknung im Ölvakuum (5 h bei Raumtemperatur) 61,43 g (Theorie: 57,58 g).

Der Kristallkuchen wurde in 80 ml heissem Methanol gelöst und zur heissen Lösung allmählich 260 ml Aceton gegeben. Die entstandene Trübung wurde durch Zugabe von 2 ml Methanol zum Verschwinden gebracht, das Gefäss verschlossen und abkühlen gelassen. Nach wenigen Minuten setzte an der Gefässwand die Kristallisation ein. Nach 48 h wurde noch 3 h im Kühlschrank (+4 °C) aufbewahrt und die Mutterlauge von der Kristallkruste abgegossen. Die Kristalle wurden mit ca. 20 ml Aceton/Methanol (1:5) und wenig Aceton gewaschen und im Vakuum getrocknet.

Es resultierten 19,33 g Kristalldrusen.

Smp. 159 °C (nach Kristallisation aus Aceton/Methanol).

$[\alpha]_D^{24} = +10,8°$ (c = 1,04 in Wasser).

Beispiel 5

Herstellung von (–)-3-Chlor-2-oxy-propyltrimethylammoniumchlorid

Zu 18,35 g (40,5 mMol) Tartrat gemäss Beispiel 1 in 65 ml Wasser wurden unter Umschwenken 4,50 g (40,5 mMol) Calciumchlorid, gelöst in 15 ml Wasser, zugetropft. Das Calciumtartrat fiel sofort kristallinisch aus. Nach 5 min wurde die Suspension im Eisbad abgekühlt (Lösung hatte pH 7) und das Calciumtartrat abgenutscht. Mit Methanol gewaschen und an der Luft getrocknet wog es 10,08 g (Theorie für das Tetrahydrat: 10,54 g, Ausbeute 95,6%).

Das Filtrat (und Wasch-Methanol) wurde im Rotationsverdampfer bei 50 °C Badetemperatur eingedampft. Der feste Rückstand wog 17,0 g (Theorie: 15,24 g) und wurde mit 25 ml absoluten Ethanol bei 70 °C digeriert. Die Suspension wurde im Eisbad gekühlt und die Kristalle abgenutscht. Nach dem Waschen mit Ethanol/Aceton = 1:1 und Aceton wurde an der Luft getrocknet.

Ausbeute: 10,24 g farblose Kristalle = 67,2% der Theorie, Smp. 214 °C.

$[\alpha]_D^{24} = -28,76°$ (c = 0,97 in Wasser).

Beispiel 6

Herstellung von (–)-Carnitinnitrilchlorid

8,61 g (45,78 mMol) des nach Beispiel 4 hergestellten Produktes in 9 ml Methanol und 1 ml Wasser wurden in einem Bad von 50–55 °C tropfenweise innert 3 min mit 3,43 g (47,0 mMol) Natriumcyanid in 8 ml Wasser versetzt. Die Reaktionslösung, die sofort trüb wurde, wurde 20 min im Bad belassen (pH 8–9) und dann mit 5,5n Salzsäure auf pH 5 gestellt (3,0 ml Säure wurden benötigt). Nach Kühlung des Ansatzes mit einem Bad von –10 °C während einiger Minuten wurde ausgefallenes Salz abfiltriert und mit eiskaltem Methanol gewaschen und getrocknet: 1,89 g Salz.

Das Filtrat wurde bei 40 °C Badtemperatur im Vakuum eingeengt. Der Rückstand, eine gelbliche feste Masse (10,6 g) wurden in 23 g heissem Methanol aufgenommen und die 40 °C warme Lösung filtriert (Entfernung von 0,60 g unlöslichem Material). Das Filtrat wurde erneut filtriert (Abtrennung von ca. 0,1 g Salz) und bis zur Klärung erwärmt. (Gewicht der Lösung 24 g) und auf 0 °C abgekühlt. Die ausgeschiedenen Kristalle wurden abgenutscht, mit wenig Methanol (–10 °C) und Ether gewaschen und getrocknet.

Ausbeute: 4,62 g fast farblose Kristalle = 56,5% der Theorie, Smp. 244 °C.

$[\alpha]^{24}_{D} = -28{,}30°$ (c = 1,06 in Wasser)

Das Produkt enthielt Ausgangsmaterial (DC).

Nach zweimaligem Umkristallisieren aus Ethanol (95%) wurden lange Nadeln erhalten, Smp. 256 °C Zers.

$[\alpha]^{24}_{D} = -25{,}9°$ (c = 1,05 in Wasser)

Beispiel 7

Herstellung von (–)-Glycidyltrimethylammoniumchlorid

((–)-N,N,N-trimethyl-oxiranemathanamine)

Zu 9,5 g (50 mMol) (–)-3-Chlor-2-oxy-propyltrimethylammoniumchlorid (99,1%), $[\alpha]^{24}_{D} = -29{,}5°$ (c = 1, $H_2O$) Smp. 212–214 °C), gelöst in 35 ml Methanol, wurde bei Raumtemperatur unter Rühren eine Lösung von 2,05 g NaOH (98%, 50 mMol) in 45 ml Methanol zugetropft. Das Gemisch wurde während 3 h gerührt, das ausgefallene NaCl (2,6 g; 89%) filtriert und mit je 2 mal 5 ml Ethanol gewaschen. Das Filtrat und das Wasch-Ethanol wurden eingedampft.

Das Rohprodukt (8,95 g, 117%) wurde in 50 ml Chloroform aufgenommen, wobei sich nach Schütteln das Produkt allmählich löste, bis auf etwas NaCl. Dieses unlösliche NaCl (0,60 g, 20%) wurde filtriert.

Nach Eindampfen des $CHCl_3$ wurden 7,6 g (99,3%) (–)-Glycidyltrimethylammonium-chlorid erhalten.

Das Produkt enthielt kein Ausgangsmaterial (DC).

Smp. 121 bis 123,5 °C

$[\alpha]^{24}_{D} = -27{,}0°$ (c = 1 in Wasser)

IR (KBr): 3440s, 3030w, 2980w, 2940w, 1630m, 1485s, 1420w, 1270w, 1150w, 1100w, 980m, 935s, 900m, 870m, 805w, 770w.

$^1$H-NMR (300 MHz, $d_6$ – DMSO):

2,69 (dd, 1H, J=5 und 3 Hz, H-C(3));

2,93 (dd, 1H, J=5 und 5 Hz, H-C(3));

3,22 (dd, 1H, J=13 und 8 Hz, H-C(1));

3,23 (s, 9H, -$N(CH_3)_3$);

3,57 (dddd, 1H, J=8/5/3 und 3 Hz, H-C(2));

4,04 (dd, 1H, J=13 und 3 Hz, H-C(1)).

Beispiel 8

Herstellung von (–)-Glycidyltrimethylammoniumchlorid

((–)-N,N,N-trimethyl-oxiranemethanamine)

Zu 9,5 g (50 mMol) (–)-3-Chlor-2-oxy-propyltrimethylammoniumchlorid (99,1%, $[\alpha]^{24}_{D} = -29{,}5°$ (c = 1, $H_2O$), Smp. 212–214 °C), gelöst in 35 ml Methanol, wurde bei Raumtemperatur unter Rühren eine Lösung von 5,8 g KOtBu (97% 50 mMol) in 20 ml Methanol zugetropft. Das Gemisch wurde während 3 h gerührt, das ausgefallene KCl (3,95 g, 105%) filtriert und mit je 2 mal 5 ml Ethanol gewaschen. Das Filtrat und das Wasch-Ethanol wurden eingedampft.

Das Rohprodukt (9,15 g, 119%) wurde in 50 ml Chloroform aufgenommen, wobei sich nach Schütteln das Produkt allmählich löste, bis auf etwas KCl. Dieses unlösliche KCl (0,05 g, Spuren) wurde filtriert.

Nach Eindampfen des $CHCl_3$ wurden 7,5 g (98%) (–)-Glycidyltrimethylammonium-chlorid erhalten.

Das Produkt enthielt kein Ausgangsmaterial (DC).

Smp. 119 bis 121 °C

$[\alpha]^{24}_{D} = -27{,}1°$ (c = 1 in Wasser).

Beispiel 9

Herstellung von L-Carnitinnitrilchlorid

Zu 10 ml MeOH wurden 4,35 g Acetoncyanhydrin (98%, 50 mMol) und 7,9 g (50 mMol) (–)-Glycidyltrimethylammonium-chlorid zugegeben. Das Gemisch wurde bei 20 bis 25 °C gerührt, bis alles gelöst wurde. Danach wurde die Lösung innerhalb einer halben Stunde auf 45 °C erwärmt und 4 h (Dünnschichtchromatogramme) bei dieser Temperatur weitergerührt (nach einer halben Stunde bei 50 °C begann das Produkt auszufallen). Das Gemisch wurde auf 20 °C abgekühlt, die weissen Kristalle abgenutscht, 3mal mit je 6 ml Aceton gewaschen und getrocknet.

Ausbeute: 7,5 g (81,6% d.Th.)

Smp 246 °C (Zers.)

$[\alpha]^{24}_{D} = -25{,}6°$ (c = 1 in Wasser)

Das Produkt war 97,3%ig (HPLC) und erhielt noch 2,4% (–)-Glycidylstrimethylammonium-chlorid. Nach Umkristallisation aus Ethanol (95%) wurden lange Nadeln erhalten.

Smp. 256 °C (Zers.)

$[\alpha]^{24}_{D} = -25{,}8°$ (c = 1 in Wasser)

**Patentansprüche**

1. Optisch aktives Di-[3-chlor-2-oxy-propyltrimethylammonium]-tartrat.

2. Di-[(–)-3-chlor-2-oxy-propyltrimethylammonium]-L-(+)-tartrat.

3. Di-[(+)-3-chlor-2-oxy-propyltrimethylammonium]-D-(–)-tartrat.

4. Verfahren zur Herstellung von optisch aktivem Di-[3-chlor-2-oxy-propyltrimethylammonium]-tartrat nach Patentanspruch 1, dadurch gekennzeichnet, dass man von racemischem 3-Chlor-2-oxy-propyltrimethylammonium-chlorid ausgeht und dieses durch Racematspaltung mit optisch aktiver Weinsäure in das gewünschte optisch aktive Di-[3-chlor-2-oxy-propyltrimethylammonium]-tartrat überführt.

5. Verfahren zur Herstellung von Di-[(–)-3-chlor-2-oxy-propyltrimethylammonium)-L-)(+)-tartrat nach Patentanspruch 2, dadurch gekennzeichnet, dass man von racemischem 3-Chlor-2-oxy-propyltrimethylammonium-chlorid ausgeht und dieses durch Racematspaltung mit L-(+)-Weinsäure in das gewünschte Di-[(–)-3-chlor-2-oxy-propyltrimethylammonium]-L-(+)-tartrat überführt.

6. Verfahren nach Patentanspruch 5, dadurch gekennzeichnet, dass man die Racematspaltung mit L-(+)-Weinsäure in Gegenwart von Trialkylamin oder mit Hilfe des Silbersalzes der L-(+)-Weinsäure durchgeführt.

7. Verfahren zur Herstellung von Di-[(–)-3-chlor-2-oxy-propyltrimethylammonium]-L-(+)-tartrat nach Patentanspruch 5, dadurch gekennzeichnet, dass man L-(+)-Weinsäure, gelöst in Wasser oder suspendiert in Alkoholen, mit Trimethylamin umsetzt und anschliessend bei Temperaturen von 10 bis 35 °C mit Epichlorhydrin in das Di-[(–)-3-chlor-2-oxy-propyltrimethylammonium]-L-(+)-tartrat überführt.

8. Verwendung von optisch aktivem Di-[3-chlor-2-oxy-propyltrimethylammonium]-tartrat nach Patentanspruch 1 zur Herstellung von optisch aktivem Carnitinnitrilchlorid, dadurch gekennzeichnet, dass man Di-[3-chlor-2-oxy-propyltrimethylammonium]-tartrat in Weinsäure und optisch aktives 3-Chlor-2-oxy-propyltrimethylammonium-chlorid spaltet und letzteres mit anorganischen Cyaniden umsetzt.

9. Verwendung von Di-[(–)-chlor-2-oxy-propyltrimethylammonium]-L-(+)-tartrat nach Patentanspruch 2 zur Herstellung von (–)-Carnitinnitrilchlorid, dadurch gekennzeichnet, dass man Di-[(–)-3-chlor-2-oxy-propyltrimethylammonium]-L-(+)-tartrat in Weinsäure und (–)-3-Chlor-2-oxy-propyltrimethylammonium-chlorid spaltet und letzteres mit anorganischen Cyaniden umsetzt.

10. Verwendung von optisch aktivem Di-[3-chlor-2-oxy-propyltrimethylammonium]-tartrat nach Patentanspruch 1 zur Herstellung von optisch aktivem Carnitinnitrilchlorid, dadurch gekennzeichnet, dass man Di-[3-chlor-2-oxy-propyltrimethylammonium]-tartrat in Weinsäure und optisch aktives 3-Chlor-2-oxy-propyltrimethylammonium-chlorid spaltet, letzteres mit starken Basen in das optisch aktive Glycidyltrimethylammonium-chlorid überführt und dieses mit Acetoncyanhydrin oder Blausäure in das optisch aktive Carnitinnitrilchlorid umsetzt.

## Claims

1. Optically active di-[3-chloro-2-oxy-propyltrimethylammonium]-tartrate.

2. Di-[(–)-3-chloro-2-oxy-propyltrimethylammonium]-L-(+)-tartrate.

3. Di-[(+)-3-chloro-2-oxy-propyltrimethylammonium]-D-(–)-tartrate.

4. Process for the production of optically active di-[3-chloro-2-oxy-propyltrimethylammonium]-tartrate according to patent claim 1, characterized in that one starts with racemic 3-chloro-2-oxy-propyltrimethylammonium-chloride and transform it into the desired optically active di-[3-chloro-2-oxy-propyltrimethylammonium]-tartrate by racemate resolution with optically active tartaric acid.

5. Process for the production of di-[(–)-3-chloro-2-oxy-propyltrimethylammonium]-L-(+)-tartrate according to patent claim 2, characterized in that one starts with racemic 3-chloro-2-oxy-propyltrimethylammonium-chloride and transform it into the desired di-[(–)-3-chloro-2-oxy-propyltrimethylammonium]-L-(+)-tartrate by racemate resolution with L-(+)-tartaric acid.

6. Process according to patent claim 5, characterized in that the racemate resolution is carried out with L-(+)-tartaric acid in the presence of trialkyl amine or by means of the silver salt of L-(+)-tartaric acid.

7. Process for the production of di-[(–)-3-chloro-2-oxy-propyltrimethylammonium]-L-(+)-tartrate according to patent claim 5, characterized in that L-(+)-tartaric acid, dissolved in water or suspended in alcohols, is reacted with trimethyl amine and subsequently transformed into di-[(–)-3-chloro-2-oxy-propyltrimethylammonium]-L-(+)-tartrate with epichlorohydrin at temperatures of 10 to 35 °C.

8. The use of optically active di-[3-chloro-2-oxy-propyltrimethylammonium]-tartrate according to patent claim 1 in the production of optically active carnitine nitrile chloride, characterized in that di-[3-chloro-2-oxy-propyltrimethylammonium]-tartrate is resolved into tartaric acid and optically active 3-chloro-2-oxy-propyltrimethylammonium-chloride and that the latter is reacted with inorganic cyanides.

9. The use of di-[(–)-3-chloro-2-oxy-propyltrimethylammonium]-L-(+)-tartrate according to patent claim 2 in the production of (–)-carnitine nitrile chloride, characterized in that di-[(–)-3-chloro-2-oxy-propyltrimethylammonium]-L-(+)-tartrate is resolved into tartaric acid and (–)-3-chloro-2-oxy-propyltrimethylammonium-chloride and that the latter is reacted with inorganic cyanides.

10. The use of optically active di-[3-chloro-2-oxy-propyltrimethylammonium]-tartrate according to patent claim 1 in the production of optically active carnitine nitrile chloride, characterized in that di-[3-chloro-2-oxy-propyltrimethylammonium]-tartrate is resolved into tartaric acid and optically active 3-chloro-2-oxy-propyltrimethylammonium-chloride, the latter is transformed into the optically active glycidyl trimethylammonium-chloride and said chloride is reacted into the optically active carnitine nitrile chloride with acetone cyanohydrin or hydrocyanic acid.

## Revendications

1. Tartrate de di-[3-chloro-2-oxy-propyltriméthylammonium] optiquement actif.

2. L-(+)-tartrate de di-[(–)-3-chloro-2-oxy-propyltriméthylammonium].

3. Di-(–)-tartrate de di-[(+)-3-chloro-2-oxy-propyltriméthylammonium].

4. Procédé pour la préparation de tartrate de di-[3-chloro-2-oxy-propyltriméthylammonium] selon la revendicaction 1, caractérisé en ce qu'on part de chlorure de 3-chloro-2-oxy-propyltriméthylammonium racémique et en ce qu'on transforme celui-ci par clivage de racémate au moyen d'acide tartrique optiquement actif, en le tartrate de di-[3-chloro-2-oxy-propyltriméthylammonium] optiquement actif désiré.

5. Procédé pour la préparation du L-(+)-tartrate de di-[(–)-3-chloro-2-oxy-propyltriméthylammonium] selon la revendication 2, caractérisé en ce qu'on part de chlorure de 3-chloro-2-oxy-propyltriméthylammonium racémique et en ce qu'on transforme celui-ci, par clivage de racémate au moyen d'acide L-(+)-tartrique, en le L-(+)-tartrate de di-[(–)-3-chloro-2-oxy-propyltriméthylammonium] désiré.

6. Procédé selon la revendication 5, caractérisé en ce qu'on effectue le clivage de racémate au moyen d'acide L-(+)-tartrique en présence de trialcoylamine ou à l'aide du sel d'argent de l'acide L-(+)-tartrique.

7. Procédé pour la préparation du L-(+)-tartrate de di-[(–)-3-chloro-2-oxy- propyltriméthylammonium] selon la revendication 5, caractérisé en ce qu'on fait réagir de l'acide L-(+)-tartrique, dissous dans l'eau ou en suspension dans des alcools, avec de la triméthylamine et en ce qu'on transforme ensuite, à des températures de 10 à 35 °C, au moyen d'épichlorhydrine, en le L-(+)-tartrate de di-[(–)-3-chloro-2-oxy-propyltriméthylammonium].

8. Utilisation du tartrate de di-[3-chloro-2-oxy-propyltriméthylammonium] optiquement actif selon la revendication 1 pour la préparation de chlorure de nitrile de carnitine optiquement actif, caractérisée en ce qu'on clive le tartrate de di-[3-chloro-2-oxy-propyltriméthylammonium] en acide tartrique et en chlorure de 3-chloro-2-oxy-propyltriméthylammonium] optiquement actif et en ce qu'on fait réagir ce dernier avec des cyanures non organiques.

9. Utilisation du L-(+)-tartrate de di-[(–)-3-chloro-2-oxy-propyltriméthylammonium] selon la revendication 2 pour la préparation de chlorure de nitrile de (–)-carnitine, caractérisée en ce qu'on clive le L-(+)-tartrate de di-[(–)-3-chloro-2-oxy-propyltriméthylammonium] en acide tartrique et chlorure de (–)-3-chloro-2-oxy-propyltriméthylammonium] et en ce qu'on fait réagir ce dernier avec des cyanures non organiques.

10. Utilisation de tartrate de di-[3-chloro-2-oxy-propyltriméthylammonium] optiquement actif selon la revendication 1 pour la préparation de chlorure de nitrile de carnitine optiquement actif, caractérisée en ce qu'on clive du tartrate de di-[3-chloro-2-oxy-propyltriméthylammonium] en acide tartrique et en chlorure de 3-chloro-2-oxy-propyltriméthylammonium] optiquement actif, en ce qu'on transforme ce dernier, au moyen de bases fortes, en le chlorure de glydicyltriméthylammonium optiquement actif et en ce qu'on fait réagir ce dernier avec de l'acétone-cyanhydrine ou de l'acide cyanhydrique pour donner le chlorure de nitrile de carnitine optiquement actif.